**Europäisches Patentamt**

**European Patent Office**

**Office européen des brevets**

(11) Veröffentlichungsnummer: **0 224 706**

**A1**

## (12) EUROPÄISCHE PATENTANMELDUNG

(21) Anmeldenummer: 86114656.1

(51) Int. Cl.⁴: **C07D 263/34**

(22) Anmeldetag: 22.10.86

(30) Priorität: 01.11.85 CH 4710/85

(43) Veröffentlichungstag der Anmeldung:
10.06.87 Patentblatt 87/24

(84) Benannte Vertragsstaaten:
**AT BE CH DE FR GB IT LI NL**

(71) Anmelder: **F. HOFFMANN-LA ROCHE & CO.
Aktiengesellschaft**

**CH-4002 Basel(CH)**

(72) Erfinder: **Nösberger, Paul, Dr.
Passwangstrasse 1
CH-4127 Birsfelden(CH)**

(74) Vertreter: **Urech, Peter, Dr. et al
Grenzacherstrasse 124 Postfach 601
CH-4002 Basel(CH)**

(54) **Verfahren zur Herstellung eines Oxazols.**

(57) Verfahren zur Herstellung von 5-Cyano-4-methyl-oxazol, dadurch gekennzeichnet, dass man ein 4-Methyl-5-nieder-alkoxycarbonyl-oxazol mit Ammoniak in der Gasphase und in Gegenwart eines Zirkonoxid-oder Hafniumoxidkatalysators umsetzt.

EP 0 224 706 A1

## Verfahren zur Herstellung eines Oxazols

Die vorliegende Erfindung betrifft ein neues Verfahren zur Herstellung von 5-Cyano-4-methyl-oxazol, einem wichtigen Zwischenprodukt bei der Herstellung von Pyridoxin (Vitamin $B_6$).

Bisher wurde dieses Oxazolderivat durch Umsetzung von 5-Carbamoyl-4-methyl-oxazol mit Phosphorpentoxid unter Erhitzen hergestellt. Dieses bekannte Verfahren ist jedoch mit gewissen Nachteilen verbunden, beispielsweise einer verhältnismässig geringen Ausbeute, die auf sehr leicht eintretende Verkohlung zurückzuführen ist.

Eine Verbesserung dieses bekannten Verfahrens besteht darin, die Umsetzung des 5-Carbamoyl-4-methyl-oxazols mit dem Phosphorpentoxid in Gegenwart eines Lösungsmittels, nämlich Chinolin, durchzuführen (vgl. US-Patentschrift Nr. 3.222.374). Auch dieses Verfahren hat Nachteile, und zwar die unter den Reaktionsbedingungen festgestellte Instabilität, den unangenehmen Geruch sowie die Gesundheitsschädlichkeit des Chinolins. Ferner sind die Verfahren zur Regenerierung des Chinolins und zur Aufarbeitung der Folgeprodukte des benötigten Phosphorpentoxids zu umweltfreundlichen Produkten aufwendig und mit einer Reihe technologischer Probleme belastet. Ausserdem sind sowohl Chinolin als auch Phosphorpentoxid teure und knappe Rohstoffe.

Ein weiteres bekanntes Verfahren zur Herstellung des 5-Cyano-4-methyl-oxazols besteht darin, das 5-Carbamoyl-4-methyl-oxazol mit einem Niederalkancarbonsäureanhydrid umzusetzen und das Reaktionsgemisch oder das aus diesem isolierte 4-Methyl-5-(N-niederalkanoyl-carbamoyl)-oxazol einer Pyrolyse zu unterwerfen. Die pyrolytische Endstufe hat jedoch gewisse Nachteile, wie insbesondere das Arbeiten bei hohen Temperaturen, Probleme mit den Reaktorwerkstoffen und die Bildung von schwer rezyklisierbaren Nebenprodukten.

Aufgabe der vorliegenden Erfindung ist daher die Schaffung eines Verfahrens, welches es ermöglicht, das 5-Cyano-4-methyl-oxazol in einfacher und billiger Weise herzustellen, wobei die oben aufgezählten Nachteile der bekannten Verfahren nicht auftreten und wobei das gewünschte 5-Cyano-4-methyl-oxazol in hoher Ausbeute und in guter Qualität anfällt. Diese Aufgabe wird gemäss der vorliegenden Erfindung dadurch gelöst, dass man von einem 4-Methyl-5-niederalkoxycarbonyl-oxazol und Ammoniak ausgeht und die Umsetzung des Ausgangsmaterials in der Gasphase und in Gegenwart eines Zirkonoxid-oder Hafniumoxidkatalysators bewerkstelligt, aber das als Zwischenprodukt gebildete 5-Carbamoyl-4-methyl-oxazol nicht isoliert und separat behandelt.

Als Niederalkoxycarbonylgruppe kommen in Frage insbesondere solche Gruppen, deren Alkoxygruppe bis zu 6 Kohlenstoffatome enthält und geradkettig oder verzweigt ist. Die Niederalkoxycarbonylgruppe ist vorzugsweise $C_{1-3}$-Alkoxy-carbonyl, insbesondere Aethoxycarbonyl.

Der Zirkonoxid-bzw. Hafniumoxidkatalysator kann in im wesentlichen reiner Form, also unverdünnt, vorliegen oder mit einem inerten Material gemischt oder auf einem Trägermaterial fixiert, also verdünnt, sein. Beispiele geeigneter inerter Materialien bzw. Trägermaterialien sind Aluminiumoxid und Aluminiumsilikat. Das bevorzugte Trägermaterial ist Aluminiumoxid. Falls der Katalysator verdünnt ist, so beträgt die Katalysatormenge zweckmässigerweise etwa 5 bis 30 Gewichtsprozent der Gesamtmenge. Vorzugsweise wird allerdings der Katalysator in unverdünnter Form verwendet. In jedem Fall beträgt die spezifische Oberfläche des unverdünnten oder verdünnten Katalysators vorzugsweise mindestens 10 m²/g. Die besten Ergebnisse werden durch Verwendung von unverdünnten Katalysatoren mit spezifischen Oberflächen im Bereich von 10 bis 40 m²/g bzw. von verdünnten Katalysatoren mit spezifischen Oberflächen im Bereich von 10 bis 90 m²/g (je nach Dichte des verwendeten Begleitmaterials) erzielt.

Die erfindungsgemäss verwendeten Katalysatoren sind zum Teil im Handel erhältlich oder können leicht in an sich bekannter Weise hergestellt werden, beispielsweise durch Fällen aus einer wässrigen Lösung von Zirkonyl-bzw. Hafniumoxychlorid mit wässrigem Ammoniak, Filtration, Waschen mit Wasser, Trocknen, Pulverisieren, Sieben, Tablettieren und Calcinieren, z.B. bei ca. 500-800°C. Falls der Katalysator verdünnt werden soll, erfolgt das Zusammenfügen mit dem inerten Material bzw. Trägermaterial auf einer beliebigen Stufe eines solchen Verfahrens, allerdings spätestens unmittelbar vor dem Tablettieren. Die Art der Herstellung und Vorbehandlung des unverdünnten oder verdünnten Katalysators beeinflusst u.a. die Grösse der spezifischen Oberfläche und damit die katalytischen Eigenschaften.

Die zweckmässige Menge des eingesetzten Katalysators kann vom Fachmann leicht ermittelt werden. Sie ist insbesondere von der Menge eingesetzter Ausgangsmaterialien, der Reaktionstemperatur und der Form und Grösse des Reaktors abhängig.

Die erfindungsgemässe Umsetzung des 4-Methyl-5-nieder-alkoxycarbonyl-oxazols mit Ammoniak erfolgt zweckmässigerweise mit einem Ueberschuss an Ammoniak, wobei das Molverhältnis Ammoniak:Oxazol mindestens 1,5:1 beträgt. Vorzugsweise beträgt dieses Verhältnis 5:1 bis 10:1.

2

Ferner erfolgt die Umsetzung zweckmässig in einem Temperaturbereich von etwa 230-400°C, vorzugsweise bei Temperaturen zwischen etwa 260°C und etwa 350°C. In bezug auf den Druck, unter dem die Umsetzung durchgeführt wird, erfolgt aus technischen und wirtschaftlichen Gründen die Umsetzung vorzugsweise unter Atmosphärendruck.

Zweckmässigerweise wird das 4-Methyl-5-niederalkoxycarbonyl-oxazol in Form einer Lösung in einem geeigneten organischen Lösungsmittel in den geheizten Reaktor eingeführt. Als Lösungsmittel eignet sich ganz besonders Toluol.

Die Umsetzung kann auch noch in Gegenwart von zugesetztem Inertgas, insbesondere Stickstoff, durchgeführt werden.

Das erfindungsgemässe Verfahren kann kontinuierlich oder in Chargen, vorzugsweise kontinuierlich, durchgeführt werden, und zwar vorteilhaft in einem Festbettreaktor, der beispielsweise aus einer oder mehreren mit dem Katalysator gepackten, thermostabilisierbaren Säulen besteht. Im Produktionsmassstab eignet sich ganz besonders ein Rohrbündel-Wärmetauscher. Der Durchmesser und die Länge der einzelnen Rohre ist nicht kritisch. Zweckmässigerweise wird der Durchmesser so gewählt, dass ein durch Koksablagerung desaktivierter Katalysator mittels eines sauerstoffhaltigen Gases, vorzugsweise Luft, regeneriert werden kann. Eine Regenerierung des Katalysators durch Abbrennen des abgelagerten Koks ist nomalerweise nach 100 bis 200 Stunden Gebrauch des Katalysators notwendig.

Dem Reaktor werden vorzugsweise 50 bis 200 g 4-Methyl-5-niederalkoxycarbonyl-oxazol/ l Katalysatorvolumen/Stunde (Durchflussgeschwindigkeit) zugeführt. Die Mengen an zugeführtem Ammoniak, Lösungsmittel und Inertgas werden vorteilhaft so gewählt, dass die Konzentration an 4-Methyl-5-niederalkoxycarbonyl-oxazol 10 Molprozent nicht überschreitet.

Das erfindungsgemässe Verfahren liefert das gewünschte 5-Cyano-4-methyl-oxazol in hoher Reinheit. Zwecks Aufarbeitung werden die gasförmigen Nebenprodukte von dem kondensierbaren Oxazol sowie dem bei der Reaktion gebildeten Wassen und Alkohol, abgetrennt, und zwar zweckmässigerweise durch Abkühlen. Die Hauptmenge der Abgase wird vorzugsweise wieder dem Reaktor zugeführt. Es wird hierauf aus dem resultierenden Zweiphasengemisch die organische Phase abgetrennt und diese einer Rektifikation unterworfen.

Das erfindungsgemässe Verfahren wird durch die folgenden Beispiele weiter veranschaulicht.

Beispiel 1

Der Reaktor besteht aus einem senkrecht aufgestellten Glasrohr (Länge ca. 30 cm, Durchmesser ca. 2 cm), welches von einem Rohrofen umgeben und mit einem Kühler verbunden ist. Der obere und untere Teil des Glasrohres ist mit Keramikkugeln, der mittlere Teil mit Katalysator (30 ml) gefüllt. Als Katalysator wird ein kommerziell erhältlicher Zirkonoxid-Katalysator (Harshaw Zr-0304T) verwendet, der vor dem Einsatz 2 Stunden bei 700°C calciniert wurde.

Die Heizung des Ofens wird auf 310°C eingestellt, und es werden dem Reaktor stündlich 18 ml einer 25%igen Lösung von 5-Aethoxycarbonyl-4-methyl-oxazol in Toluol. 7 l Ammoniak und 7 l Stickstoff zugeführt. Die Reaktionsprodukte werden in einem Kühler kondensiert und gaschromatographisch analysiert.

Bei einem Umsatz von 99% beträgt die Ausbeute an 5-Cyano-4-methyl-oxazol 78%.

Beispiel 2  ·

In dem in Beispiel 1 beschriebenen Reaktor wird als Katalysator ein kommerziell erhältliches Zirkonoxidpulver (Dynazirkon® F der Firma Dynamit Nobel), das vor dem Einsatz mit Stearinsäure (1 Gewichtsprozent) als Schmiermittel tablettiert (3x3 mm) und anschliessend 3 Stunden bei 700°C calciniert wurde, eingesetzt.

Unter Verwendung dieses Katalysators beträgt, bei einem Umsatz von 99%, die Ausbeute an 5-Cyano-4-methyl-oxazol 75%.

Beispiel 3

In dem in Beispiel 1 beschriebenen Reaktor wird als Katalysator ein aus Zirkonnitrat hergestelltes Zirkonoxid eingesetzt. Bei einem Umsatz von 96% beträgt die Ausbeute an 5-Cyano-4-methyl-oxazol 74%.

Der verwendete Katalysator wird wie folgt hergestellt:

Zu einer gerührten Lösung von 500 g Zirkonnitrat-pentahydrat in 2,5 l destilliertem Wasser gibt man 15 g mikrokristalline Cellulose (Avicel PH 101) und 310 ml 25%iges wässriges Ammoniak. Der Niederschlag wird abgenutscht, mit destilliertem Wasser gewaschen und während 3 Stunden bei 700°C calciniert. Dann wird das resultierende pulverisierte Zirkonoxid mit 4 Gewichtsprozent Stearinsäure (Schmiermittel) vermischt, tablettiert (Durchmesser 6 mm, Höhe 3 mm) und während 3 Stunden bei 500°C ausgeheizt.

Beispiel 4

In dem in Beispiel 1 beschriebenen Reaktor wird als Katalysator ein aus Zirkonylchlorid hergestelltes Zirkonoxid eingesetzt. Bei einem Umsatz von 99% beträgt die Ausbeute an 5-Cyano-4-methyl-oxazol 81%.

Der verwendete Katalysator wird wie folgt hergestellt:

Zu einer gerührten Lösung von 397 g Zirkonylchlorid-hexahydrat in 1,1 l destilliertem Wasser gibt man 25 g Graphit und 200 g 25%iges wässriges Ammoniak. Der Niederschlag wird abgenutscht, mit destilliertem Wasser gewaschen, während 16 Stunden bei 130°C getrocknet, tablettiert (Durchmesser 3 mm, Höhe 2,2 mm) und während 3 Stunden bei 700°C calciniert.

Beispiel 5

In dem in Beispiel 1 beschriebenen Reaktor wird ein auf Aluminiumoxid aufgetragener Zirkonoxid-Katalysator eingesetzt und die Temperatur des Ofens auf 320°C eingestellt. Bei einem Umsatz von 98% beträgt die Ausbeute an 5-Cyano-4-methyl-oxazol 76%.

Der verwendete Katalysator wird wie folgt hergestellt:

70 g $\gamma$-Aluminiumoxid (Harshaw Al-111-73R) werden während 16 Stunden bei 1000°C ausgeheizt und dann mit 40,5 g Zirkonnitrat-pentahydrat in 40,5 ml destilliertem Wasser imprägniert. Nach der Imprägnierung wird in einem Rohrofen bei einem Luftstrom von 200 l/Stunde innerhalb von 5 Stunden auf 450°C geheizt und anschliessend bei dieser Temperatur gehalten, bis keine nitrosen Gase mehr auftreten. Die Belegung des Zirkonoxids auf dem so hergestellten Katalysator beträgt 15%.

Beispiel 6

In dem in Beispiel 1 beschriebenen Reaktor wird ein aus Hafniumoxychlorid hergestellter Hafniumoxid-Katalysator eingesetzt. Bei einem Umsatz von 98% beträgt die Ausbeute an 5-Cyano-4-methyl-oxazol 82%.

Der verwendete Katalysator wird wie folgt hergestellt:

Zu einer gerührten Lösung von 500 g Hafniumoxychlorid-octahydrat in 1,4 l destilliertem Wasser gibt man 200 g 25%iges wässriges Ammoniak. Der Niederschlag wird abgenutscht, mit destilliertem Wasser gewaschen, während 16 Stunden bei 130°C getrocknet, mit 4 g Stearinsäure vermischt, tablettiert und während 3 Stunden bei 600°C calciniert.

Beispiel 7

In dem in Beispiel 1 beschriebenen Reaktor wird ein auf Aluminiumsilikat aufgetragener Zirkonoxid-Katalysator eingesetzt und die Temperatur des Ofens auf 350°C eingestellt. Bei einem Umsatz von 98% beträgt die Ausbeute an 5-Cyano-4-methyl-oxazol ca. 65%.

Der verwendete Katalysator wird wie in Beispiel 5 beschrieben hergestellt, jedoch unter Verwendung eines Aluminiumsilikat-Trägers mit einer spezifischen Oberfläche von ca. 30 m²/g (Norton SA 3232) anstelle des Aluminiumoxid-Trägers. Die Belegung des Zirkonoxids auf dem so hergestellten Katalysator beträgt in diesem Fall 9,3%.

0 224 706

Beispiel 8

In dem in Beispiel 1 beschriebenen Reaktor wird ein aus Zirkonpropylat hergestellter Zirkonoxid-Katalysator eingesetzt. Bei einem Umsatz von 99% beträgt die Ausbeute an 5-Cyano-4-methyl-oxazol ca. 81%.

Der verwendete Katalysator wird wie folgt hergestellt:

Zu einer gerührten Lösung von 525 g Zirkonpropylat in 650 ml Aethanol gibt man 325 ml destilliertes Wasser. Anschliessend wird das resultierende Gemisch am Rotationsverdampfer eingedampft und der Rückstand während 16 Stunden bei 150°C getrocknet. Das resultierende pulverförmige Zirkonoxid wird mit 8 g Stearinsäure vermischt, tablettiert und 3 Stunden bei 700°C calciniert.

Beispiele 9-14

Das in Beispiel 1 beschriebene Verfahren wird wiederholt, jedoch mit verschiedenen Ammoniak-und Stickstoff-Durchflussgeschwindigkeiten. Die Ergebnisse dieser Versuche, aus denen hervorgeht, wie die Ausbeute von diesen Durchflussgeschwindigkeiten abhängt, werden in der nachstehenden Tabelle zusammengestellt:

| Beispiel | Ammoniak-Durchfluss-geschwindigkeit (1/Stunde) | Stickstoff-Durchfluss-geschwindigkeit (1/Stunde) | Umsatz (%) | Ausbeute (%) |
|---|---|---|---|---|
| 9 | 7 | 14 | 99 | 74 |
| 10 | 7 | – | 99 | 65 |
| 11 | 3,5 | 14 | 95 | 74 |
| 12 | 3,5 | – | 99 | 30 |
| 13 | 1,8 | 7 | 98 | 73 |
| 14 | 0,9 | 7 | 99 | 73 |

**Ansprüche**

1. Verfahren zur Herstellung von 5-Cyano-4-methyl-oxazol, dadurch gekennzeichnet, dass man ein 4-Methyl-5-niederalkoxycarbonyl-oxazol mit Ammoniak in der Gasphase und in Gegenwart eines Zirkonoxid- oder Hafniumoxidkatalysators umsetzt.

2. Verfahren nach Anspruch 1, dadurch gekennzeichnet, dass man als 4-Methyl-5-niederalkoxycarbonyl-oxazol 5-Aethoxycarbonyl-4-methyl-oxazol verwendet.

3. Verfahren nach Anspruch 1 oder 2, dadurch gekennzeichnet, dass die spezifische Oberfläche des unverdünnten oder verdünnten Katalysators mindestens 10 m²/g beträgt.

4. Verfahren nach einem der Ansprüche 1 bis 3, dadurch gekennzeichnet, dass bei der Umsetzung das Molverhältnis Ammoniak:4-Methyl-5-niederalkoxycarbonyl-oxazol mindestens 1,5:1 beträgt.

5. Verfahren nach Anspruch 4, dadurch gekennzeichnet, dass das Molverhältnis 5:1 bis 10:1 ist.

6. Verfahren nach einem der Ansprüche 1 bis 5, dadurch gekennzeichnet, dass die Umsetzung in einem Temperaturbereich von 230°C bis 400°C erfolgt.

7. Verfahren nach Anspruch 6, dadurch gekennzeichnet, dass im Temperaturbereich von 260°C bis 350°C gearbeitet wird.

8. Verfahren nach einem der Ansprüche 1 bis 7, dadurch gekennzeichnet, dass das 4-Methyl-5-niederalkoxycarbonyl-oxazol in Form einer Lösung in Toluol in den geheizten Reaktor eingeführt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 010 697 (F. HOFFMANN-LA ROCHE UND CO. AG) * Anspruch 1 * | 1 | C 07 D 263/34 |
| A | DE-A-2 616 349 (F. HOFFMANN-LA ROCHE UND CO. AG) * Anspruch 1 * | 1 | |
| A | DE-A-2 535 310 (F. HOFFMANN-LA ROCHE UND CO. AG) * Anspruch 1 * | 1 | |
| D,A | US-A-3 222 374 (G.O. CHASE) * Anspruch 1 * | 1 | |
| A | US-A-2 203 861 (A.G. DEEM et al.) * Ansprüche 1, 2 * | 1,6,7 | RECHERCHIERTE SACHGEBIETE (Int. Cl.4) |
| A | EP-A-0 072 401 (CHEMISCHE WERKE HÜLS AG) * Ansprüche * | 1,6,7 | C 07 C 120/08 C 07 D 263/34 |

Der vorliegende Recherchenbericht wurde für alle Patentansprüche erstellt.

| Recherchenort | Abschlußdatum der Recherche | Prüfer |
|---|---|---|
| BERLIN | 22-01-1987 | HASS C V F |